Europäisches Patentamt

European Patent Office

Office européen des brevets

(19)

(11) Numéro de publication: **0 011 005**
**B1**

(12)

# FASCICULE DE BREVET EUROPEEN

(21) Numéro de dépôt: **79400722.9**

(22) Date de dépôt: **08.10.79**

(51) Int. Cl.³: **G 21 C 17/02, G 01 N 1/12**

(54) Tête de prélèvement d'agent de refroidissement dans un réacteur nucléaire.

(30) Priorité: **27.10.78 FR 7830643**

(43) Date de publication de la demande:
**14.05.80 Bulletin 80/10**

(45) Mention de la délivrance du brevet:
**18.03.81 Bulletin 81/11**

(84) Etats Contractants Désignés:
**DE GB IT NL**

(56) Documents cités:
**DE - A - 2 521 833**
**FR - A - 2 020 129**
**GB - A - 1 466 071**

(73) Titulaire: **NOVATOME**
**20 Avenue Edouard Herriot**
**F-92350 Le Plessis Robinson (FR)**

(72) Inventeur: **Deverger, Christian**
**3 rue Jean Mermoz**
**F-78000 Versailles (FR)**
(72) Inventeur: **Le Baud, Patrice**
**12 rue St Méderic**
**F-78000 Versailles (FR)**
(72) Inventeur: **Perotto, Gérard**
**17 résidence les Rieux**
**F-91120 Palaiseau (FR)**

(74) Mandataire: **Bouget, Lucien et al,**
**CREUSOT-LOIRE 15 rue Pasquier**
**F-75383 Paris Cedex 08 (FR)**

Courier Press, Leamington Spa, England.

# Tête de prélèvement d'agent de refroidissement dans un réacteur nucléaire

L'invention concerne une tête de prélèvement d'agent de refroidissement dans un réacteur nucléaire à des fins d'analyse, associée pour le prélèvement dans la cuve du réacteur et pour le dépôt de l'agent de refroidissement prélevé, dans un dispositif de désaccouplement et de transport, à la partie inférieure d'une perche de prélèvement verticale.

Dans les réacteurs nucléaires, tels que les réacteurs à neutrons rapides refroidis par du sodium liquide constituant le fluide primaire du réacteur nucléaire, il est nécessaire de prélever à certains moments, de petites quantités de sodium liquide dans le réacteur nucléaire, pour effectuer sur ces échantillons de sodium prélevés dans le réacteur, des analyses pour déterminer l'état du fluide de refroidissement.

Cette opération de prélèvement à l'intérieur de la cuve du réacteur nucléaire présente des difficultés car le sodium est à haute température et contaminé par des substances radioactives.

D'autre part le prélèvement doit se faire à l'intérieur de la cuve à une profondeur relativement importante en-dessous de la dalle de cette cuve et dans une ambiance radioactive.

Pour effectuer ces prélèvements d'échantillons, on utilise une perche de très grande longueur à l'extrémité inférieure de laquelle on fixe une capsule de prélèvement par vissage sur un embout fileté. Cet embout fileté est usiné à l'extrémité de la partie inférieure de la perche de prélèvement qui comporte une partie supérieure et une partie inférieure réunies entre elles par un moyen de liaison démontable.

La perche est donc constituée de trois parties: une partie supérieure de très grande longueur, une partie inférieure beaucoup plus courte et enfin une tête de prélèvement vissée sur la partie inférieure.

La tête de prélevement comprend elle-même deux éléments solidaires l'un de l'autre: une capsule de prélèvement et un dispositif de raccordement de la capsule et de la perche.

La capsule comprend un réceptacle pour le sodium, des ouvertures faisant communiquer ce réceptacle avec le milieu dans lequel on plonge l'extrémité inférieure de la perche et une partie externe profilée destinée à assurer le blocage en rotation de la tête de prélèvement, lorsque cette tête de prélèvement est engagée dans un socle de désaccouplement de la tête et de la perche comportant une partie concave de forme correspondante à la partie externe profilée de la tête de prélèvement.

Pour le prélèvement, on fait pénétrer la perche par une ouverture prévue dans la dalle de la cuve du réacteur au-dessus de laquelle on a disposé un château de transport destiné à recueillir l'échantillon de sodium pour son transport vers le socle de désaccouplement dans lequel la tête de prélèvement est séparée de la perche. Le socle de désaccouplement dans lequel la capsule a été déposée peut servir également au transport de cette capsule vers le laboratoire d'analyse des produits radio-actifs, lorsque le château de transport a été déposé sur le socle de désaccouplement qui constitue alors le fond de ce château de transport.

Lorsque le château de transport est en position de service audessus de la cuve du réacteur, il est relié à un dispositif de jonction permettant d'isoler l'intérieur du réacteur par rapport à l'atmosphère extérieure, tout en permettant le passage étanche de la perche grâce à un ensemble de joints d'étanchéité. La perche peut donc se déplacer dans la direction verticale pour le prélèvement et le retrait de l'échantillon indépendamment du dispositif de jonction et du château de plomb, la partie inférieure de la perche de prélèvement à laquelle est reliée le tête de prélèvement pouvant alors être séparée de le partie supérieure de cette perche pour être transportée vers le socle de désaccouplement. Pendant ce transport la perche de prélèvement a été remontée de telle sorte que la tête de prélèvement se trouve au niveau du château de transport.

Un système de vannes permet également d'isoler l'intérieur du réacteur, lorsque la perche de prélèvement a été remontée avec l'échantillon de sodium liquide.

Lorsqu'on vient placer le château de transport renfermant un échantillon de sodium dans la tête de prélèvement, sur le socle de désaccouplement, un dispositif de jonction permet également une jonction étanche entre le château de transport et le socle de désaccouplement.

Le socle de désaccouplement comprend généralement à sa partie supérieure une ouverture de form hexagonale permettant l'engagement de la partie inférieure de la capsule de prélèvement de forme correspondante pour son blocage en rotation. Il suffit alors de faire tourner la partie inférieure de la perche de prélèvement solidaire de la tête de prélèvement pour dévisser cette tête comportant la capsule et la séparer de la perche de prélèvement.

Cependant, il est très souvent difficile, sinon impossible, de dévisser la capsule lorsque celle-ci a été vissée à fond de filet sur l'extrémité inférieure de la perche.

Le but de l'invention est donc de proposer une tête de prélèvement d'agent de refroidissement dans un réacteur nucléaire à des fins d'analyse, associée à la partie inférieure d'une perche de prélèvement verticale pour le prélèvement dans la cuve du réacteur et pour le dépôt de l'agent de refroidissement prélevé dans un socle de désaccouplement et de transport et constituée d'une capsule de prélèvement et d'un dispositif de raccordement démontable de la capsule et de la perche, la capsule de prélèvement comportant de façon connue un réceptacle, des ouvertures mettant en communication le réceptacle avec le milieu dans lequel est plongée la partie inférieure de la perche et une zone externe profilée pour l'engagement de la capsule dans un dispositif antagoniste de blocage en rotation de cette capsule par rapport à la perche, lors du dépôt du réceptacle dans le socle de désaccouplement avant transport, cette tête de prélèvement pouvant être assemblée à la perche de façon que la liaison soit extrêmement résistante au moment du prélèvement du sodium liquide et de façon que la séparation de la capsule de prélèvement et de la perche dans le socle de désaccouplement associé au château de transport se fasse de façon certaine, au moment où l'on dépose la capsule dans le socle de désaccouplement.

Dans ce but, la tête de prélèvement comporte entre la capsule de prélèvement et le dispositif de raccordement, une zone de rupture en torsion résistant à la flexion, pour la séparation par un effort de torsion sur la partie inférieure de la perche, de la capsule par rapport au dispositif de raccordement, lorsque la capsule est bloquée en rotation.

On va maintenant décrire, en se reportant aux figures jointes en annexe, un mode de réalisation d'une tête de prélèvement suivant l'invention associée à une perche de prélèvement, par comparaison avec le dispositif utilisé jusqu'ici pour le prélèvement de sodium dans un réacteur à neutrons rapides.

La figure 1 représente dans une vue en coupe par un plan vertical, une perche de prélèvement munie d'une capsule telle qu'utilisée jusqu'ici, en position dans une cuve d'un réacteur nucléaire refroidi par du sodium liquide.

La figure 2 représente dans une vue en coupe analogue à celle de la figure 1, la partie inférieure d'une perche de prélèvement équipée d'une tête de prélèvement possédant une zone de rupture en torsion, suivant l'invention.

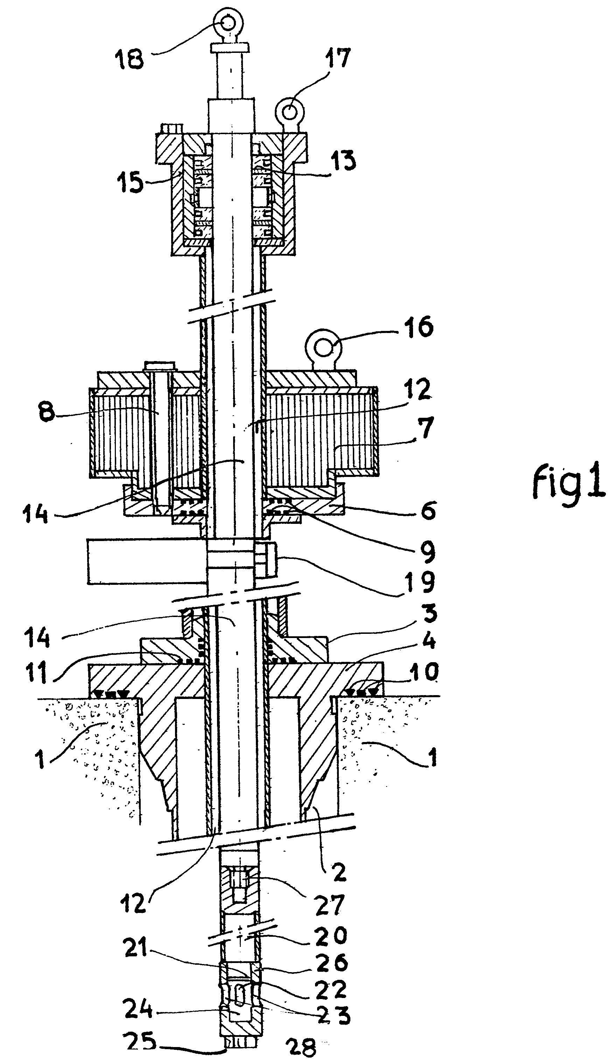

Sur la figure 1, on voit la dalle 1 d'un réacteur nucléaire à neutrons rapides à l'intérieur de laquelle a été prévue une traversée 2 pour le dispositif de prélèvement de sodium liquide. Les pièces d'étanchéité et de fixation 3 et 4 fixées sur la dalle 1 soutiennent un support 6 sur lequel vient reposer un château de plomb 7 fixé sur le support 6 par des tiges telles que 8, l'étanchéité entre le support et le château de plomb étant assurée par des joints 9.

Des joints 10 et 11 permettent également d'isoler l'intérieur du réacteur de l'ambiance extérieure au niveau des pièces de jonction 3 et 4.

Le château de transport, son support et les pièces de jonction sont percés à leur partie centrale de façon à constituer un puits cylindrique vertical 12 à l'intérieur duquel peut se déplacer la perche 14.

Au-dessus du château de transport, un passage étanche 15 muni de joints circulaires 13 permet un déplacement vertical de la tige dans un sens ou dans l'autre tout en maintenant isolé le puits central 12 communiquant avec l'intérieur du réacteur, de l'ambiance extérieure.

Le château de transport 7 et le dispositif d'étanchéité 15 comportent des anneaux de levage tels que 16 et 17 permettant le levage de ce château de plomb avec le dispositif d'étanchéité lorsque la perche de prélèvement 14 est remontée de façon que sa partie inférieure, constituée par la tête de prélèvement, soit au niveau du château de plomb.

La perche de prélèvement 14 comporte à sa partie supérieure un anneau de levage 18 permettant le déplacement dans la direction verticale de la perche de levage par rapport au château de transport 7 et au dispositif d'étanchéité 15.

Un ou plusieurs dispositifs de levage permettent d'une part de déplacer verticalement la perche de prélèvement à l'intérieur du puits 12 et de la maintenir en position haute ou dans une position intermédiaire quelconque et d'autre part de soulever l'ensemble constitué par le château de transport, le dispositif d'étanchéite et la partie inférieure de la perche de prélèvement, lorsque les tiges de fixation du château de transport ont été désolidarisées du support 6, pour emmener la partie inférieure de la perche munie de sa capsule de prélèvement, le château et le dispositif d'étanchéité vers un socle de désaccouplement comportant une pièce d'appui pour la mise en place du château de transport et l'introduction de la partie inférieure de la perche de prélèvement dans le dispositif de désaccouplement.

La pièce de jonction 3 comporte également un dispositif obturateur 19 permettant d'isoler l'intérieur de la cuve du réacteur lorsque le château de transport n'est pas disposé au-dessus de la cuve.

La partie inférieure de la perche de prélèvement est constituée par un tube 20 à l'extrémité inférieure duquel est fixée une pièce d'assemblage 21 filetée sur une partie de sa longueur pour la fixation de la tête de prélèvement 22, par l'intermédiaire d'un manchon taraudé 26 permettant l'assemblage acec la pièce 21 et constituant le dispositif de raccordement de la tête de prélèvement.

La capsule de prélèvement 28 solidaire de cette pièce de raccordement 26 comporte des ouvertures 23 par lesquelles pénètre le sodium liquide lorsque la perche de prélèvement est plongée dans le fluide primaire du réacteur. En dessous des ouvertures 23, la partie interne de la capsule constitue le réceptacle 24 de métal liquide.

La surface inférieure 25 de la capsule 28 présente une form prismatique à base hexagonale que permet d'engager la capsule dans une forme correspondante dans le socle de désaccouplement, pour le dévissage de la tête de prélèvement et sa séparation de la perche.

Le tube 20 constituant la partie inférieure de la perche de prélèvement est solidaire à sa partie supérieure d'un embout taraudé 27 permettant l'assemblage de la partie inférieure de la perche de prélèvement à la partie supérieure 14 de cette perche de prélèvement par l'intermédiaire d'une extrémité filetée cooperant avec l'embout taraudé 27.

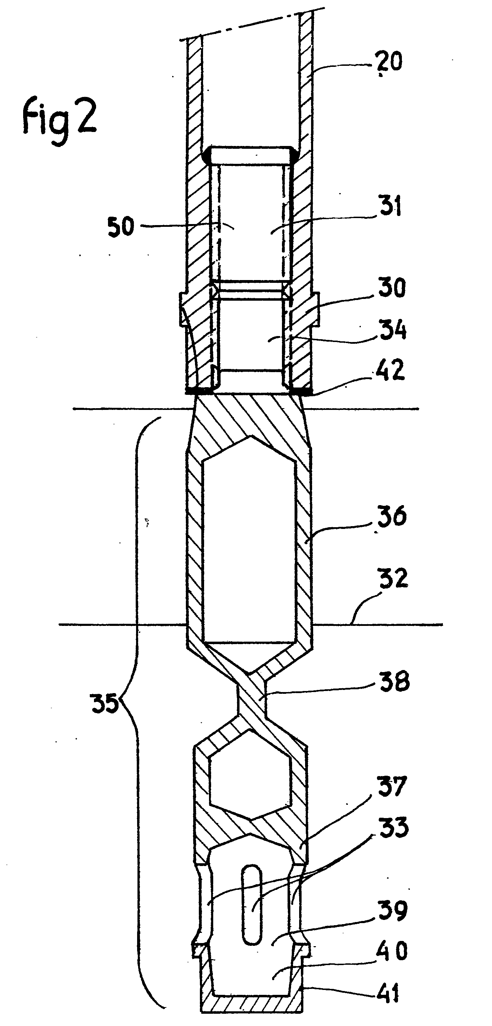

On va maintenant se reporter à la figure 2 sur laquelle est représentée la partie inférieure du tube 20 d'une perche de prélèvement en tout point semblable à celle qui vient d'être décrite.

Cette perche de prélèvement est équipée à sa partie inférieure d'une tête de prélèvement suivant l'invention.

A l'extrémité du tube 20 est soudée une pièce creuse 30 comportant un alésage central 31 taraudé sur toute sa hauteur.

La tête de prélèvement 35 comportant une partie supérieure 36 constituant le dispositif de raccordement entre la capsule et la perche et une partie inférieure 37 constituant la capsule de prélèvement proprement dite est vissée à l'intérieur de la pièce 30 par l'intermédiaire de la partie supérieure filetée 34 du dispositif de raccordement 36 de cette tête de prélèvement 35. La partie filetée 34 de la pièce 36 a une longueur inférieure à la longueur de l'alésage 31, si bien qu'il est nécessaire de visser un bouchon 50 dans la partie supérieure de l'alésage pour éviter d'avoir un volume vide dans l'alésage 31. Le bouchon 50 est soudé à sa partie supérieure sur la pièce 30 pour assurer l'étanchéité de la jonction entre les pièces 50 et 30. Cependant, il serait également possible de prévoir une partie filetée 34 de plus grande longueur occupant pratiquement tout le volume de l'alésage 31.

La partie supérieure 36 de la tête de prélèvement est réunie à la partie inférieure 37 de cette tête de prélèvement constituant la capsule par l'intermédiaire d'une partie cylindrique de faible diamètre 38 constituant une zone de rupture susceptible d'être cisaillée sous un effort de torsion mais résistant aux efforts de flexion qui peuvent être appliqués par l'intermédiaire de le perche de prélèvement.

Le diamètre de la zone de cisaillement est beaucoup plus petit que celui de la partie filetée de raccordement des pièces 34 et 30.

Il est d'ailleurs déterminé pour que le couple de rupture soit inférieur au couple normal de serrage des deux parties filetées.

La capsule de prélèvement 37 comporte des ouvertures 33 mettant en communication le fluide du réacteur, lorsque la perche est en position de prélèvement dans cette cuve, avec la partie interne 39 de la capsule constituant en-dessous des ouvertures 33, un réceptacle de prélèvement 40.

On a représenté sur la figure 2 le niveau du sodium liquide dans le réacteur à l'intérieur duquel plonge la capsule de prélèvement. Ce niveau 32 lorsque la perche de prélèvement est dans sa position la plus basse, atteint la partie inférieure du dispositif de raccordement 36 de la tête de prélèvement.

De cette façon le sodium liquide n'est jamais en contact avec la zone de raccordement par vissage entre la tête de prélèvement et la perche au niveau des pièces 30 et 34, la sécurité de l'assemblage étant d'autre part assurée grâce à une rondelle 42. De cette façon, le montage de la tête de prélèvement par rapport à la perche deprélèvement est toujours réalisable dans de bonnes conditions.

La surface externe 41 de la capsule au niveau du réceptacle 40, est usinée en forme de prisme à base hexagonale de sorte que, lorsque la tête de prélèvement est disposée, après prélèvement du sodium liquide dans la cuve du réacteur, dans le socle de désaccouplement et de transport des échantillons, cette partie prismatique 41 vient s'engager dans une ouverture de forme correspondante ménagée dans le socle de désaccouplement. De cette façon, la capsule est maintenue bloquée en rotation si bien que, lorsqu'on exerce un effort de torsion sur la perche de prélèvement, la zone de rupture 38 subit un cisaillement suffisant pour séparer la capsule 37 comportant le réceptacle de sodium de la partie 36 de raccordement de cette capsule qui reste fixée à la perche de prélèvement.

On va maintenant décrire brièvement la fonctionnement du dispositif selon l'invention dans le cas du prélèvement du sodium liquide dans une cuve du réacteur nucléaire et du dépôt de la capsule de prélèvement sur un socle de désaccouplement permettant également son transport.

On transporte l'ensemble constitué par le château de transport et la perche de prélèvement, grâce à un organe de manutention, sur le support correspondant disposé au-dessus d'une ouverture de la dalle du réacteur nucléaire et l'on assemble le château de transport par l'intermédiaire des tiges de fixation 8 sur ce support, en effectuant l'étanchéité par rapport à l'atmosphère extérieure grâce au joint tel que 9.

La perche de prélèvement est alors en position haute dans le château de plomb et après différents balayages en argon et ouverture de l'obturateur 19, on descend cette perche de prélèvement jusqu'à sa position la plus basse où la capsule de prélèvement est dans la position représentée à la figure 2 sous le niveau du sodium liquide.

Le sodium liquide vient remplir le réceptacle 40 grâce aux ouvertures 33 ménagées dans la partie inférieure de la capsule. On remonte alors la perche de prélèvement à l'intérieur du château de transport dans sa position haute grâce au dispositif de levage soulevant la perche par son anneau 18 et l'on sépare la partie supérieure de la perche de prélèvement de la partie inférieure sur laquelle est fixée la tête de prélèvement, grâce au dispositif de vissage 27.

On referme les vannes d'étanchéité et l'on sépare le château de plomb de son support avant de le transporter grâce au dispositif de manutention, par l'intermédiaire de ses anneaux 16 vers un socle de désaccouplement et de transport sur lequel on le met en position sur un support à proximité du point de prélèvement afin que la surface prismatique 41 de la capsule de prélèvement vienne en prise avec l'évidement prévu à la partie supérieure du socle de désaccouplement. Pour cela, le socle de désaccouplement comporte un bouchon vertical à sa partie centrale, ce bouchon venant se loger dan le canal central du château de transport lorsque le château est placé sur le socle et supportant à sa partie supérieure la forme prismatique femelle dans laquelle vient s'engager la surface 41 de la capsule.

On exerce alors un mouvement de torsion dans l'axe du la perche sur l'extrémité supérieure de la partie inférieure de la perche de prélèvement et la zone de rupture 38 permet la séparation par cisaillement du dispositif de jonction 36 par rapport à la capsule de prélèvement 37. On peut alors sortir entièrement la partie inférieure de la perche de prélèvement à laquelle est resté fixé le dispositif de raccordement 36, du dispositif de transport et refermer la partie supérieure de ce dernier par un couvercle bouchon venant se placer sur le château de transport. Il est alors possible d'emmener l'échantillon de sodium refroidi et solidifié à l'intérieur du dispositif de transport, vers le laboratoire d'analyse. Pour être remise en êtat, la partie inférieure de la perche de prélèvement est d'abord décontaminée, puis la partie de raccordement 36 est dévissée après libération de la rondelle-frein 42. Un ensemble neuf 35 complet est revissé et freiné par une nouvelle rondelle-frein 42.

On voit que les avantages principaux de l'invention sont que l'assemblage entre la perche de prélèvement et la capsule est extrêmement résistant et évite tout risque de perte de la capsule dans le réacteur au moment du prélèvement et que d'autre part, la séparation entre les deux parties de la tête de prélèvement pour le dépôt de la capsule dans le socle de désaccouplement et de transport se faite de façon sûre.

D'autre part les pièces d'assemblage par vissage entre la tête de prélèvement et la perche ne sont jamais en contact avec le sodium liquide pendant les opérations de prélèvement dans la cuve du réacteur. Enfin lors des manipulations de la perche de prélèvement, la capsule ne peut pas se séparer du dispositif de raccordement à la perche de prélèvement, si la capsule vient heurter un obstacle car la zone 38 ne se rompt pas en flexion alors que la séparation est possible par torsion de la zone 38.

Mais l'invention ne se limite pas au mode de réalisation qui vient d'être décrit, elle en comporte au contraire toutes les variantes et l'on peut imaginer des modifications de points de détail sans pour autant sortir du cadre de l'invention.

En particulier, le dispositif selon l'invention s'applique dans tous les cas où un réacteur nucléaire est refroidi par un réfrigérant liquide, bien que l'application préférentielle de ce dispositif se situe dans le domaine des réacteurs à neutrons rapides refroidis au sodium liquide.

## Revendications

1. Tête de prélèvement d'agent de refroidissement liquide dans un réacteur nucléaire, à des fins d'analyse, associée à la partie inférieure d'une perche de prélèvement verticale, pour le prélèvement dans la cuve du réacteur et pour le dépôt de l'agent de refroidissement prélevé dans un socle de désaccouplement et de transport et constituée d'une capsule de prélèvement (37) et d'un dispositif de raccordement démontable de la capsule et de la perche, la capsule de prélèvement comportant de façon connue un réceptacle (39) du liquide prélevé, des ouvertures (33) mettant en communication le réceptacle avec le milieu dans lequel est plongée la partie inférieure de la perche et une zone externe profilée (41) pour l'engagement de la capsule dans un dispositif antagoniste de blocage en rotation de cette capsule, lors du dépôt du réceptacle dans le socle de désaccouplement, par rapport à la perche, avant transport, caractérisée par le fait qu'elle comporte, entre la capsule de prélèvement (37) et le dispositif de raccordement (36), une zone de rupture en torsion (38) résistant à le flexion, pour la séparation par un effort de torsion sur la partie inférieure de la perche (20), de la capsule (37) par rapport au dispositif de raccordement (36), lorsque la capsule (37) est bloquée en rotation.

2. Tête de prélèvement selon la revendication 1, caractérisée par le faite que le dispositif de raccordement (36) de la capsule (37) et de la perche (20) comporte une extrémité filetée (34) permettant sa fixation par vissage dans un alésage taraudé (31) aménagé dans la partie inférieure de la perche de prélèvement (20), le diamètre de l'extrémité filetée (34) du dispositif de raccordement (36) et de l'alésage étant suffisamment important par rapport au diamètre de la zone de rupture en torsion (28) pour que le couple de serrage normal de l'assemblage (31—34) soit supérieur au couple de cisaillement de la zone de rupture (38).

3. Tête de prélèvement suivant la revendication 2, caractérisée par le fait que la longueur de l'alésage taraudé (31) est sensiblement égale à la longueur de la partie mâle filetée (34) qu'il reçoit.

4. Tête de prélèvement suivant l'une quelconque des revendications 2 et 3, caractérisée par le fait que la zone de raccordement de la tête (35) et de la perche (20) est, lors du prélèvement, au-dessus du niveau (32) de l'agent de refroidissement liquide dans le réacteur.

## Claims

1. Head for sampling liquid cooling agent in a nuclear reactor, for the purpose of analysis, associated with the lower part of a vertical sampling pole, for taking samples in the reactor vessel and for putting the sampled cooling agent in an uncoupling and transportation stand and constituted by a sampling capsule (37) and a coupling device which can be removed from the capsule and the pole, the sampling capsule comprising in a known way a receptacle (39) for the sampled liquid, apertures (33) putting the receptacle in communication with the medium in which the lower part of the pole is immersed and an outer region (41) shaped for engaging the capsule in an opposing device for locking this capsule while rotating, when the receptacle is put in the uncoupling stand, with respect to the pole, before transportation, characterised by the fact that, between the sampling capsule (37) and the coupling device (36), it has a region (38) for breaking by torsion, resistant to bending, for separating, by a torque on the lower part of the pole (20), the capsule (37) with respect to the coupling device (36), when the capsule (37) is locked while rotating.

2. Sampling head according to claim 1, characterised by the fact that the device (36) for coupling the capsule (37) and the pole (20) has a threaded end (34) allowing it to be fixed by screwing in a screw-threaded bore (31) disposed in the lower part of the sampling pole (20), the diameter of the threaded end (34) of the coupling device (36) and the bore being large enough with respect to the diameter of the region (28) for breaking by torsion for the normal tightening torque of the assembly (31—34) to be greater than the shearing torque of the breaking region (38).

3. Sampling head according to claim 2, characterised by the fact that the length of the screw-threaded bore (31) is substantially the same as the length of the threaded male part (34) which it receives.

4. Sampling head according to either of claims 2 and 3, characterised by the fact that the region for coupling the head (35) and the pole (20) is above the level (32) of the liquid cooling agent in the reactor on sampling.

## Patentansprüche

1. Kopf zur Entnahme eines flüssigen Kühlmittels aus einem Kernreaktor zwecks Analyse, der am unteren Teil mit einem senkrechten Entnahmestab zur Entnahme aus dem Reaktorbehälter und zum Absetzen des entnommenen Kühlmittels in einen Abkuppelund Fördersockel verbunden ist und aus einer Entnahmekapsel (37) und einer von Kapsel und Stab abnehmbaren Anschlussvorrichtung besteht, wobei die Entnahmekapsel bekannterweise ein Behältnis (39) für die entnommene Flüssigkeit, Offnungen (33), durch die das Behältnis mit dem Medium kommuniziert, in das der untere Stabteil getauch wird, und eine äussere profilierte Zone (41) aufweist zum Einführen der Kapsel in eine Gegenvorrichtung zum Sperren der Drehbewegung dieser Kapsel beim Absetzen des Behältnisses in den Abkuppelsockel, im Verhältnis zum Stab, vor Fördern, dadurch gekennzeichnet, dass er zwischen Entnahmekapsel (37) und Anschlussvorrichtung (36) eine biegefeste Drehbruchzone (38) aufweist zwecks Trennung der Kapsel (37) von der Anschlussvorrichtung (36) durch eine Drehbeanspruchung am unteren Stabteil (20), wenn die Drehbewegung der Kapsel (37) gesperrt ist.

2. Entnahmekopf nach Anspruch 1 dadurch gekennzeichnet, dass die Anschlussvorrichtung (36) der Kapsel (37) und des Stabs (20) ein Gewindeende (34) aufweist zum Einschrauben in eine Gewindebohrung (31) im unteren Teil des Entnahmestabs (20), wobei der Durchmesser dieses Gewindeendes (34) der Anschlussvorrichtung (36) und der Bohrung im Vergleich zum Durchmesser der Drehbruchzone (28) genügend gross ist, damit das normale Anziehmoment der Verbindung (31—34) grösser ist als das Schermoment der Drehbruchzone (38).

3. Entnahmekopf nach Anspruch 2 dadurch gekennzeichnet, dass die Länge der Gewindebohrung (31) ziemlich der Länge des von ihr aufgenommenen Gewindeteils (34) entspricht.

4. Entnahmekopf nach einem der Ansprüche 2 und 3 dadurch gekennzeichnet, dass die Anschlusszone des Kopfs (35) und des Stabs (20) bei der Entnahme sich oberhalb des Standes (32) des flüssigen Kühlmittels im Reaktor befindet.

fig1

fig 2